# EUROPEAN PATENT APPLICATION

(11) **EP 3 744 837 A1**
(43) Date of publication of application: **02.12.2020**
(21) Application number: 19744369.0
(22) Date of filing: 24.01.2019
(51) Int. Cl.: C12N 9/04, C12N 1/20, C12P 17/02

(54) **METHOD FOR PROMOTING SYNTHESIS OF SORBITOL DEHYDROGENASE AND COENZYME PYRROLOQUINOLINE FROM GLUCONOBACTER OXYDANS**

(30) Priority: 26.01.2018 CN 201810078913
(71) Applicant: Zhejiang University of Technology, Hangzhou, Zhejiang 310014 (CN); ZheJiang Medicine Co., Ltd. Xinchang Pharmaceutical Factory, Shaoxing, Zhejiang 312500 (CN)
(72) Inventor: ZHENG, Yuguo, Hangzhou, Zhejiang 310014 (CN); KE, Xia, Hangzhou, Zhejiang 310014 (CN); LU, Yanghui, Hangzhou, Zhejiang 310014 (CN); HU, Zhongce, Hangzhou, Zhejiang 310014 (CN); WU, Yang, Shaoxing, Zhejiang 312500 (CN)
(74) Representative: Pallini Gervasi, Diego
(86) International application number: PCT/CN2019/073044
(87) International publication number: WO 2019/144920

(57) **Abstract**

The present invention discloses a method for promoting the fermentation of *Gluconobacter oxydans* to produce D-sorbitol dehydrogenase and pyrroloquinoline quinone. The method comprises: *Gluconobacter oxydans* is inoculated to a fermentation culture medium, fermented and cultured under the conditions of 28-32 °C and 150-180 rpm for 6-24 hours, the fermented solution is centrifuged, and wet bacteria are collected, thus acquiring bacteria cells containing D-sorbitol dehydrogenase and pyrroloquinoline quinone. The method promotes the synthesis of coenzyme pQQ and the enzyme activity of per unit volume D-sorbitol dehydogenase, *Gluconobacter oxydans* cultured and acquired using the method is biotransformed to synthesize miglitol precursor 6-deoxy-6-amino(N-hydroxyethyl)-α- L-furan sorbose (6NSL), the conversion progress of the product 6NSL is increased by 21-35%, and a biotransformation step cycle is reduced from 48 hours to 36 hours. In addition, under a same substrate concentration (60 g/L), the cumulative concentration of the product 6NSL is increased by 10 g/L or more.

## Description

### FIELD OF THE INVENTION

The present invention relates to fields of fermentation engineering and biotransformation, in particular, relates to the fermentation cultivation of *Gluconobacter oxydans* and the preparation of cell membrane D-sorbitol dehydrogenase (SLDH) and coenzyme pQQ, and uses of the strain in the biotransformation and synthesis of miglitol precursor 6-deoxy-6-amino (N-hydroxyethyl)-α- L-furan sorbose (6NSL).

### BACKGROUND OF THE INVENTION

Miglitol[(2R,3R,4R,5S)-2-hydroxymethyl-1-(2-hydroxyethyl)-3,4,5-piperidinetriol, Miglitol] as hypoglycemic drugs is developed by Bayer company. Miglitol is used for reducing absorption of sugars by inhibiting α-glucosidase activity of small intestinal mucosal epidermal cells, and has the advantages of safety, effectiveness, good tolerance and little toxic side effect.

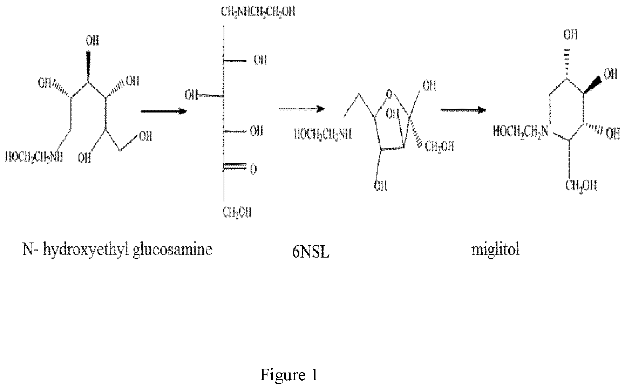

Current common method of synthesizing miglitol is a chemical biological grouping method. The key catalytic step of the method requires that the substrate N- hydroxyethyl glucosamine is subjected to selective asymmetric oxidation at 4-position hydroxyl by using D-sorbitol dehydrogenase (EC 1.1.99.22) located on the cells membrane of *Gluconobacter oxydans* under synergistic action of coenzyme Pyrroloquinoline quinone (pQQ), to obtain an intermediate and then directly coupled chemical hydrogenation cyclization synthesis to form miglitol (refer to Figure 1). The method simplifies production processes, improves product yields and reduces synthesis costs. The key step in the production process of miglitol is a biocatalytic synthesis of the intermediate 6NSL, comprising two steps: the first step is to fermentate *Gluconobacter oxydans* to produce high activity sorbitol dehydrogenase; the second step is to synthsize miglitol precursor 6NSL by using resting cells catalysting N-hydroxyethyl glucosamine. In addition, *Gluconobacter oxydans* has been widely used for catalytic synthesis of dihydroxyacetone and selective oxidation catalytic synthesis process of vitamin C and other polyols, based on incomplete oxidation and high efficiency catalytic properties of sorbitol dehydrogenase (SLDH).

The Chinese patent publication No. CN 101302549A discloses the process of producing miglitol by using resting cells prepared by *Gluconobacter oxydans* cultivatd by a culture medium comprises sorbitol, yeast extract. However the mass percentage of yeast extracts is up to 2.4wt.%, and fermentation costs are higher. In addition, US patent No. US4806650, US5401645 etc. have disclosed a process for synthesizing miglitol by using N-hydroxyethyl glucosamine as substrate by way of a chemical biological grouping method. But the process lacks of key parameters directly affecting a biotransformation ability of miglitol during fermentation, for example, enzyme activities of per unit volume membrane sorbitol dehydogenase and determination and monitoring of coenzyme pQQ content. According to characteristics of producing sorbitol dehydrogenase by fermentation of *Gluconobacter oxydans,* the present invention firstly proposed that adding sodium glutamate and partial replacing yeast extract can effectively promote synthesis of Coenzyme pQQ; moreover, can increase fermentation volume and enzyme activity of sorbitol dehydrogenase; and finally can effectively increase space-time yield of 6NSL obtained from the conversion of bacteria per unit fermentation volume, and can reduce production cost of miglitol. In addition, applying the fermentation culture medium formulation is to obtain high activity *Gluconobacter oxydans,* incomplete oxidation of sorbitol dehydrogenase is not only limited to the synthesis of miglitol, but also other polyols such as dihydroxyacetone, and catalytic synthesis of key chiral compounds such as vitamin C.

### SUMMARY OF THE INVENTION

In order to solve some limiting factors such as high proportion and high cost of yeast extract of fermentation culture medium in the process of fermentative production of *Gluconobacter oxydans* resting cells, the present invention proposes a cheap and efficient fermentation culture medium, and adds small amount of sodium glutamate for replacing yeast extract so that it not only reduces fermentation costs but also further increases fermentation volume and enzyme activity of sorbitol dehydrogenase, and provides a good basis for subsequent resting cell catalyzed synthesis of miglitol intermediate 6NSL, on basis of fermentation metabolism characteristics of *Gluconobacter oxydans* and pathway of synthesis and metabolism of membrane sorbitol dehydrogenase and its coenzyme pQQ.

In order to achieve the above purpose, the present invention provides a technical solution as follows:
A method for promoting the fermentation of *Gluconobacter oxydans* to produce D-sorbitol dehydrogenase (SLDH) and pyrroloquinoline quinone (pQQ) proposed by the present invention comprises: *Gluconobacter oxydans* is inoculated to a fermentation culture medium, fermented and cultured under the conditions of 28-32 °C and 150-180 rpm for 6-24 hours, a fermented solution is centrifuged, and wet bacteria are collected, thus acquiring bacteria cells containing D-sorbitol dehydrogenase and pyrroloquinoline quinone; the fermentation culture medium comprises D-sorbitol 50-80g/L, yeast extract 0.5-30g/L, KH₂PO₄ 5g/L, K₂HPO₄ 5g/L, amino acid 0.2-2g/L, deionized water as solvent, pH 6.5.

Further, amino acid in the fermentation culture medium is preferably sodium glutamate, leucine, tyrosine or proline. Preferably, the concentration of the amino acid in the fermentation culture medium is 0.5-1g/L, more preferably 1g/L.

Further, the amino acid is preferably sodium glutamate, and adding sodium glutamate to the fermentation culture medium in the fermentation process and partially replacing the yeast extract.

Further, a concentration of the yeast extract is 1-7 g/L, more preferably 5 g/L.

Further, the fermentation culture medium comprises D-sorbitol 50g/L, yeast extract 5g/L, KH₂PO₄ 5g/L, K₂HPO₄ 5g/L, sodium glutamate 1g/L, deionized water as solvent, pH 6.5.

Further, the *Gluconobacter oxydans* is subjected to bevel activated culture and seed culture before fermental cultivation, and then a seed solution at an inoculated dose of concentration of 5-15% by volume is inoculated to the fermentation culture medium to be fermented.

Further, the bevel activated culture is to inoculate *Gluconobacter oxydans* to a slant medium, and cultivate in a constant temperature incubator at 28 °C for 3-5 days. It will start with inoculating when CaCO3 in the slant medium becomes transparent and bright and no bacteria are detected by visual inspection, and then obtains a slanted bacterium. The slant medium comprises yeast extract 2-10g/L, calcium carbonate 5-20g/L, glucose 10-60g/L, agar 24g/L, deionized water as solvent, the pH value is natural; preferably, the slant medium comprises yeast extract 5 g/L, calcium carbonate 5g/L, glucose 50g/L, agar 24g/L, deionized water as solvent, pH value is natural.

Further, the seed culture is to inoculate the slanted bacterium to a seed culture medium, and shaking bottle fermental cultivation under a condition of 28°C and 225rpm for 24-48 hours to obtain a seed solution. OD₆₀₀ ≥ 8 meets a transfer standard. It appears by microscopic examination that the bacteria are short and rod-shaped, deeply staining and no bacteria. The seed culture medium comprises D-sorbitol 30-60g/L, yeast extract 10-30g/L, KH₂PO₄ 1-5g/L, K₂HPO₄ 0.1-1 g/L, deionized water as solvent, pH4.0-7.0. Preferably, the seed culture medium comprises D-sorbitol 50g/L, yeast extract 20g/L, KH₂PO₄ 5g/L, K₂HPO₄ 5g/L, deionized water as solvent, pH6.5.

Further, *Gluconobacter oxydans* is preferably *Gluconobacter oxydans* CCTCC No. M208069 preserved by the Chinese Typical Culture Preservation Center (CCTCC), the Chinese patent publication number is CN101591681A.

In the fermental cultivation process of agitated reactor, a relatively saturated dissolved oxygen needs to be controlled at 5%-30%. The fermentation is terminated when the stirring speed is reduced and sorbitol in the culture medium is almost exhausted. The fermentation period is controlled at 14-16 hours. It appears by microscopic examination of fermentation telophase cells that the bacteria are short and rod-shaped, deeply staining and no bacteria.

The present invention also relates to collection and cleaning of bacteria in the preparation of *Gluconobacter oxydans* resting cells. In particular, *Glucondans oxydans* obtained from the fermentation is filtered and collected by a ceramic microfiltration membrane; washing the bacteria with ultrapure water in the filtration process until the filtrate becomes colorless and transparent.

*Gluconobacter oxydans* resting cells fermentation-prepared by the present invention is used for catalyzing and producing miglitol precursor 6NSL. The preferred process includes the following step: using N-hydroxyethyl glucosamine as a substrate, adding MgSO₄·7H₂O, and afterwards adding resting cells (measured by wet bacteria weight), adding deionized water as a reaction medium, adjusting pH to 4.5-5.5, to form a reaction system; carrying out a conversion reaction at 5-30 °C, 400-600rpm for 36h; to acquire a conversion reaction solution to obtain 6NSL. In the reaction system, the final concentration of the substrate is 40-100g/L, the final concentration of MgSO₄·7H₂O is 1-5 g/L, and the final concentration of the resting cells is 20-100 g/L.

In comparison with the prior art, the advantageous effect of the present invention is mainly embodied in the following aspects.

The present invention is directed to fermentation characteristics of *Gluconobacter oxydans* and catalytic characteristics of membrane-localized D-sorbitol dehydrogenase, significantly reduce production cost of *Gluconobacter oxide* fermenting and producing enzymes by adding small amount of sodium glutamate to the fermentation culture medium and partially replacing of components of yeast extract. On this basis the above content, the method further promotes synthesis of coenzyme pQQ and enzyme activity of D-sorbitol dehydrogenase per unit volume. *Gluconobacter oxydans* obtained by the method is used for biotransformation synthesis of Migltol precursor 6NSL. Consequently it increases the conversion process of 6NSL to 21-35%, shortens the biological transformation step cycle, and also shortens the resting cell transformation process from 48 hours to 36 hours. In addition, the cumulative concentration of the product 6NSL is increased to more than 10g/L, under the same substrate concentration (60g/L). The proposal of the present invention effectively reduces the production cost of the biocatalytic synthesis of 6NSL in the key section of the chemical biocombination process of the miglitol production and also effectively improves the space-time yield of the miglitol precursor 6NSL, and lays a foundation for industrial mass producation of miglitol.

### DESCRIPTION OF DRAWINGS

Figure 1 is a synthetic route of miglitol by a chemical and biological group method.

### DETAILED DESCRIPTION AND PREFERRED EMBODIMENTS THEREOF

Hereafter, the present invention will be described specifically with reference to the examples. The examples are given only for illustration of the technical solution of the present invention. However, the high-activity D-sorbitol dehydrogenase and its coenzyme pQQ obtained from resting cells of *Gluconobacter oxydans* cultured in the fermentation culture medium provided by the present invention are not only used for the synthesis of miglitol precursor 6NSL, but also used for the selective oxidation catalysis process of other polyols.

### Example 1

*Gluconobacter oxydans* (CCTCC No. M 208069, Chinese Patent Publication No. CN101591681A) is inoculated to a slant medium, and cultivated in a constant temperature incubator at 28°C for 3-5 days to obtain a slanted bacterium. The slant medium comprise: yeast Extract 5g/L, calcium carbonate 5g/L, glucose 50g/L, agar 24g/L, deionized water as solvent, the pH value is natural.

The slanted bacterium are inoculated to a seed culture medium, and shaking cultivated at 28 ° C, 225 rpm for 48 hours to obtain a seed solution of *Gluconobacter oxydans* CCTCC No. M 208069; the seed culture medium comprises: D-sorbitol 50g/L, yeast extract 20g/L, KH₂PO₄ 5g/L, K₂HPO₄ 5g/L, and deionized water as solvent, and the pH value is 6.5.

### Example 2: Effect of amino acid species in fermentation culture medium on enzyme activity of Gluconobacter oxydans and SLDH.

Preparing a fermentation culture medium with different components is to investigate effects of different amino acids on fermental cultivation of *Gluconobacter oxydans* to synthesize of sorbitol dehydrogenase. The fermentation culture medium formulation with different amino acids comprise: D-sorbitol 50g/L, yeast extract 20g/L, KH₂PO₄ 5g/L, K₂HPO₄ 5g/L, 1g/L (sodium glutamate, leucine, tyrosine, proline), deionized water as solvent, pH6.5. No amino acids are added as a blank control group under the same conditions.

A seed solution of *Gluconobacter oxydans* CCTCC No. M 208069 of Example 1 is inoculated to a fermentation culture medium at a volume concentration of 2%, fermented at 28 ° C, 150 rpm for 24 h, and centrifuge a fermentation broth, and obtain bacterial cells for detecting a relative activity of sorbitol dehydrogenation SLDH.

Definition of enzyme activity units is referred to as an amount of enzyme to catalyze d-sorbitol and produce 1.0 µmoL L-sorbitose per minute, in a phosphate buffer system at 30°C, pH6.0 under 20 g/L sorbitol concentration. Concentrations of D-sorbitol and L-sorbose are determined by the High Performance Liquid Chromatography (HPLC).

**Operation Procedure of Enzyme Activity Detection of SLDH:** Take 20mL of a fermentation broth and centrifuge it at 10000 rpm for 10 min, discard a supernatant, wash it once, and then discard the supernatant after centrifuging again. Prepare a buffer 100 mM PBS. And afterwards dissolve 20 g/L sorbitol and 5 g/L magnesium sulfate heptahydrate to the buffer. Adjust pH to 6.0 again to establish a 10mL reaction system at 28 °C for 30min. Take 1mL sample to centrifuge for liquid phase detecting and analyzing conversion rate of sorbitol and yield of sorbitol product. Conditions of liquid phase detection are as follows: a mobile phase is 5mM H₂SO₄ solution; a flow rate is 0.6 mL/min; an injection volume is 20 µL; a column temperature is 60 °C, a time is 12 min.

The determination results are shown in Table 1.

**Table 1 Effects of different amino acids on producing SLDH by adding Gluconobacter oxydans**

| Amino acid species | Relative enzyme activity of SLDH (%) |
|---|---|
| Blank control | 100 |
| Sodium glutamate | 121 |
| Leucine | 112 |
| Tyrosine | 101 |
| Proline | 102 |

**Analysis of Results:** As shown in Table 1, in comparison with without adding amino acids (the blank control group), its relative enzyme activities of Sorbitol dehydrogenase per unit volume have different degree changes after adding 1g/L amino acid with different types to a culture medium and shaking bottle fermental cultivation for 24 hours. In particular, the enzyme activity of SLDH is almost unchanged after adding tyrosine or proline, compared with the blank control group. The enzyme activity of SLDH is respectively increased to 21% and 12% after adding sodium glutamate or leucine, compared with the blank control group, and the increase in sodium glutamate is greater. It shows that the addition of 1g/L sodium glutamate can increase the relative activity of sorbitol dehydrogenase per unit volume by more than 20%, compared with a blank control group.

### Example 3: Effect of adding sodium glutamate on fermental cultivation of Gluconobacter oxydans to synthesize coenzyme pQQ and SLDH.

Preparing a fermentation culture medium with different components is to investigate effects of sodium glutamate partially replacing yeast extract on fermental cultivation of *Gluconobacter oxydans* to synthesize sorbitol dehydrogenase and coenzyme pQQ. A fermentation culture medium formulation with different nitrogen sources comprises: D-sorbitol 50g/L, yeast extract (30 g/L, 20 g/L, 15 g/L, 10 g/L, 5 g/L), KH₂PO₄ 5g/L, K₂HPO₄ 5g/L, sodium glutamate 1g/L, deionized water as solvent, pH6.5. No sodium glutamate is added as a blank control group under the same conditions.

The seed solution of *Gluconobacter oxydans* CCTCC No. M 208069 of Example 1 is inoculated to the fermentation culture medium at a volume concentration of 2%, fermented at 28 °C, 150 rpm for 24 h, and then centrifuge a fermentation broth to obtain a supernatant. The supernatant is used for detecting the relative cumulative concentration of pQQ. The bacteric cells are used for detecting the relative activity of SLDH. Wherein the former is determined by the method of glucose dehydrogenase recombinase, and the latter is determined by the method of High Performance Liquid Chromatography (HPLC). The results are shown in Table 2.

**Table 2. Effects of addition of sodium glutamate on synthesis of pQQ and activity of SLDH under different yeast extract concentrations**

| Concentration of Yeast Extract (g/L) | Relative cumulative concentration of pQQ (%) | | Relative enzyme activity of SLDH (%) | |
|---|---|---|---|---|
| | No sodium glutamate | Adding sodium glutamate (1g/L) | No sodium glutamate | Adding sodium glutamate (1g/L) |
| 30 | 100 | 132 | 100 | 115 |
| 20 | 108 | 142 | 105 | 125 |
| 15 | 105 | 142 | 95 | 123 |
| 10 | 95 | 135 | 86 | 115 |
| 5 | 85 | 130 | 81 | 113 |

**Analysis of Results:** As shown in Table 2, different conditions of adding 1g/L sodium glutamate to the fermentation culture medium with different concentration yeast extracts are compared, after shaking bottle fermental cultivation for 24 hours. The concentration of pQQ and the coenzyme activity of sorbitol dehydrogenase per unit volume in the supernatant are significantly improved compared with the blank control group after fermental cultivating *Gluconobacter oxydans* for 24 hours. When the concentration of yeast extract is reduced to 5g/L, adding 1g/L sodium glutamate could ensure the content of coenzyme pQQ of the supernatant fermented is increased by more than 20%, compared with conventional culture medium. And the coenzyme activity of sorbitol dehydrogenase per unit volume is increased by more than 10%, compared with conventional culture medium. Consequently it effectively reduces the specific proportion of the yeast extract in the fermentation culture medium, and also reduces cost of fermentation.

### Example 4: Effect of adding sodium glutamate on fermental cultivation of Gluconobacter oxydans to synthesize coenzyme pQQ and SLDH in different time periods

Preparing a fermentation culture medium with different components is to investigate effects of adding sodium glutamate on fermental cultivation of *Gluconobacter oxydans* to synthesize coenzyme pQQ and activity of SLDH in different time periods. The fermentation culture medium formulation comprises: D-sorbitol 50g/L, yeast extract 5g/L, KH₂PO₄ 5g/L, K₂HPO₄ 5g/L, sodium glutamate 1g/L, deionized water as solvent, the pH is natural. No sodium glutamate is added as a blank control group under the same conditions.

The seed solution of *Gluconobacter oxydans* CCTCC No. M 208069 of Example 1 is inoculated to the fermentation culture medium at a volume concentration of 2%, fermented at 28 °C, 150 rpm for 24 h. Wherein Samples are taken every 6 hours, centrifuge, and detect the relative content of pQQ and the relative enzyme activity of SLDH. The results are shown in Table 3.

**Table 3 Accumulation of COENZYME PQQ and SLDH with culture time in fermentation culture medium**

| Fermentation time (H) | Relative cumulative concentration of pQQ (%) | | Relative enzyme activity of SLDH(%) | |
|---|---|---|---|---|
| | no sodium glutamate | adding sodium glutamate (1g/L) | no sodium glutamate | adding sodium glutamate (1g/L) |
| 6 | 100 | 133 | 100 | 113 |
| 12 | 266 | 402 | 125 | 152 |
| 18 | 330 | 471 | 160 | 201 |
| 24 | 351 | 534 | 137 | 177 |

**Analysis of Results:** As shown in Table 3, comparing different condition of adding 1g/L sodium glutamate to the fermentation culture medium in different time periods after shaking bottle fermental cultivation for 24 hours. The content of coenzyme pQQ in the fermentation supernatant and the enzyme activity of sorbitol dehydrogenase per unit volume have significantly improved compared with the blank control group. When the concentration of yeast extract is 5g/L, adding 1g/L sodium glutamate could increase a synthetic amount of coenzyme PQQ by more than 30% in different time periods compared with the blank control group, in order to ensure the enzyme activity of sorbitol dehydrogenase per unit volume increased by more than 10% in different time periods compared with the blank control group.

### Example 5: Comparison of the activity of 6NSL catalyzed and synthesized by resting cells of Gluconobacter oxydans obtained by adding sodium glutamate to the culture medium

Preparing a fermentation culture medium with different components is to investigate effects of sodium glutamate partially replacing yeast extract on fermental cultivation of *Gluconobacter oxydans* to synthesize sorbitol dehydrogenase and coenzyme pQQ. A fermentation culture medium formulation with different nitrogen sources comprises: D-sorbitol 50g/L, yeast extract (30 g/L, 20 g/L, 15 g/L, 10 g/L, 5 g/L), KH₂PO₄ 5g/L, K₂HPO₄ 5g/L, sodium glutamate 1g/L, deionized water as solvent, the pH is natural. No sodium glutamate is added as a blank control group under the same conditions.

The seed solution of *Gluconobacter oxydans* CCTCC No. M 208069 of Example 1 is inoculated to a fermentation culture medium at a volume concentration of 2%, fermented at 28 °C, 150 rpm for 24 h. Centrifuge a fermentation broth at 10000 rpm for 10 min, discard a supernatant, and then collect resting cells of *Gluconobacter oxydans,* and afterwards wash bacteria cells with equal volume of clean water, re-centrifuge at 10000 rpm for 10 min, discard a supernatant, collect cells to catalyze and synthesize 6NSL.

**Conversion System** (total volume: 50mL): Set a baffle shake bottle 50mL/500mL, and then add a concentration of 80g/L N-hydroxyethyl glucosamine, a concentration of 5g/LMgSO₄.7H2O, 75g/L wet bacteria, deionized water as a reaction medium to establish a catalytic reaction system. The specific reaction conditions are as follows: a temperature 15 °C; a rotation speed 220 rpm. Adjust an alkaline with 2M NaOH to maintain pH=4.5-5.0 of the conversion liquid. After transformation for 24 hours, the transformation is stopped. Take a transformation solution and then centrifuge at 10,000 rpm for 10 min. And afterwards take a supernatant and detect it by the HPLC method. The cumulative concentration of 6NSL is shown in Table 4.

**Table 4 Comparison of cumulative concentrations of 6NSL catalyzed and synthesized by resting cells of Gluconobacter oxydans obtained from fermentation with addition of sodium glutamate**

| Concentration of Yeast Extract (g/ L) | Cumulative concentration of 6NSL (g/L) | |
|---|---|---|
| | No sodium glutamate | Add sodium glutamate (1g/L) |
| 30 | 45.8 | 55.4 |
| 20 | 48.1 | 58.6 |
| 15 | 43.5 | 55.9 |
| 10 | 41.2 | 54.0 |
| 5 | 37.5 | 53.6 |

**Analysis of Results:** As shown in Table 4, adding 1g/L of sodium glutamate to the fermentation culture medium with different yeast extracts is compared. After fermental cultivating *Gluconobacter oxydans* for 24 hours, a cumulative concentration of miglitol precursor 6NSL synthesized by *Gluconobacter oxydans* resting cells resting cells catalyzing a substrate N-hydroxyethyl glucosaminehas has different degrees of improvement, comparing with the blank control group. Under a unit volume, when a concentration of yeast extract is reduced to 5g/L, the cumulative concentration of 6NSL catalyzed and synthesized by *Gluconobacter oxydans* resting cells fermented by adding a culture medium of 1g/L sodium glutamate is increased by more than 10%, comparing with cumulative concentrations of 6NSL without adding sodium glutamate, wherein, the concentration of yeast extract is 5-30g/L. And at the same time, the cumulative concentration having 53.6g/L of 6NSL under additions of a concentration 5g/L of yeast extract is higher than that of the accumulation concentration having 45.8g/L under additions of a concentration 30g/L of yeast extract. The results suggest that adding 1g/L of cheap sodium glutamate can effectively replace yeast extracts in the fermentation culture medium, and effectively reduces fermentation cost under the premise of ensuring the efficient transformation ability of the bacteria.

## Claims

1. A method for promoting the fermentation of *Gluconobacter oxydans* to produce D-sorbitol dehydrogenase and pyrroloquinoline quinone, the method comprising: *Gluconobacter oxydans* is inoculated to a fermentation culture medium, fermented and cultured under the conditions of 28-32 °C and 150-180 rpm for 6-24 hours, a fermented solution is centrifuged, and wet bacteria are collected, thus acquiring bacteria cells containing D-sorbitol dehydrogenase and pyrroloquinoline quinone; the fermentation culture medium comprises D-sorbitol 50-80g/L, yeast extract 0.5-30g/L, KH₂PO₄ 5g/L, K₂HPO₄ 5g/L, amino acid 0.2-2g/L, deionized water as solvent, pH 6.5.

2. The method according to claim 1, wherein the amino acid in the fermentation culture medium is sodium glutamate, leucine, tyrosine or proline.

3. The method according to claim 2, wherein a concentration of the amino acid in the fermentation culture medium is 0.5- 1g/L.

4. The method according to claim 2, wherein the amino acid in the fermentation culture medium is sodium glutamate.

5. The method according to claim 1, wherein a concentration of the yeast extract is 1-7g/L.

6. The method according to claim 1, wherein the fermentation culture medium comprises D-sorbitol 50g/L, yeast extract 5g/L, KH₂PO₄ 5g/L, K₂HPO₄ 5g/L, sodium glutamate 1g/L, deionized water as solvent, pH 6.5.

7. The method according to claim 1, wherein *Gluconobacter oxydans* is subjected to bevel activated culture and seed culture before fermental cultivation, and then a seed solution at an inoculated dose of concentration of 5-15% by volume is inoculated to the fermentation culture medium to be fermented.

8. The method according to claim 7, wherein the bevel activated culture is to inoculate *Gluconobacter oxydans* to a slant medium, and cultivate in a constant temperature incubator at 28 °C for 3-5 days to obtain a slanted bacterium; the slant medium comprises yeast extract 2-10g/L, calcium carbonate 5-20g/L, glucose 10-60g/L, agar 24g/L, deionized water as solvent, the pH value is natural.

9. The method according to claim 8, wherein the seed culture is to inoculate the slanted bacterium to a seed culture medium, and then shaking bottle fermental cultivate under the conditions of 28°C and 225-235 rpm for 24-48 hours to obtain a seed solution; the seed culture medium comprises D-sorbitol 30-60g/L, yeast extract 10-30g/L, KH₂PO₄ 1-5g/L, K₂HPO₄ 0.1-1 g/L, deionized water as solvent, pH4.0-7.0 .

10. The method according to claim 1, wherein *Gluconobacter oxydans* is *Gluconobacter oxydans* CCTCC No. M 208069.
